# EUROPÄISCHE PATENTSCHRIFT

(11) **EP 1 593 434 B1**
(45) Veröffentlichungstag und Bekanntmachung des Hinweises auf die Patenterteilung: **23.07.2008**
(21) Anmeldenummer: 05009896.1
(22) Anmeldetag: 06.05.2005
(51) Int. Cl.: B01L 3/00, G01N 33/52, B31D 1/02, G01N 33/487

(54) **Verfahren zur Herstellung eines Analysebandes für Flüssigproben**
Method for manufacturing a test strip for fluid samples
Procédé de fabrication d'une bandelette d'analyse d'échantillons liquides

(30) Priorität: 07.05.2004 DE 102004024041
(43) Veröffentlichungstag der Anmeldung: 09.11.2005
(73) Patentinhaber: F. Hoffmann-La Roche AG, 4070 Basel (CH); Roche Diagnostics GmbH, 68305 Mannheim (DE)
(72) Erfinder: Zimmer, Volker, 69221 Dossenheim (DE); Hoenes, Joachim, 64673 Zwingenberg (DE); Ruhl, Werner, 67117 Limburgerhof (DE)
(74) Vertreter: Pfiz, Thomas

(56) Entgegenhaltungen:
- EP-A- 0 299 517
- WO-A-02/100274
- GB-A- 1 073 596
- US-A- 4 218 421
- US-A- 4 328 057
- US-A- 4 452 887
- US-B1- 6 357 503

## Beschreibung

Die Erfindung betrifft ein Verfahren gemäß dem Oberbegriff des unabhängigen Patentanspruchs.

Solche Analysebänder lassen sich vor allem zur Blutzuckerbestimmung in tragbaren Testgeräten einsetzen, mit denen auch von Laien die erforderlichen Untersuchungsschritte einfach und schnell vorgenommen werden können. Anstelle herkömmlicher einzelner Teststreifen sind auf dem spulenförmig aufgewickelten Analyseband eine Vielzahl von mit einer geeigneten Testchemie versehenen Testfeldem fortlaufend angeordnet. Die Körperflüssigkeit wird dabei auf einem durch Bandvorlauf in eine aktive Position gebrachten Testfeld appliziert, um sodann einen geräteintemen Nachweis beispielsweise durch eine optische Messeinheit zu ermöglichen. Auf diese Weise lassen sich eine Vielzahl von Tests durchführen, ohne dass eine separate Handhabung und Entsorgung disposibler Teststreifen erforderlich wäre.

In der US-5,077,010 ist ein Analyseband in Form einer Bandkassette beschrieben. Die im Abstand voneinander befindlichen Testfelder sind aus einer Reagenzschicht und einer Spreitschicht für die Untersuchungsflüssigkeit gebildet. Über die Art der Verbindung der Testfelder mit dem Trägerband und die Bildung der Zwischenräume ist in dieser Druckschrift nichts ausgesagt.

In der WO 02/100274 ist ein Analyseband offenbart. Auf dem Analyseband werden Testbereiche in gewissem Abstand voneinander unter Blistern angeordnet.

In der US 4,475,969 ist ein Verfahren zur Herstellung eines Etikettenbands offenbart. Der Abstand zwischen den Etiketten wird bestimmt durch das Übertragen von selbstklebenden Testetiketten von einem ersten Transportband auf ein zweites schneller laufendes Transportband.

In der US 2003/0211619 ist ein Verfahren zur Herstellung eines Anaysebands offenbart. Die einzele Testbereiche werden durch Pick-and-Place Geräte auf das Analyseband übertragen.

Ausgehend hiervon liegt der Erfindung die Aufgabe zugrunde, ein Verfahren und eine Vorrichtung sowie ein geeignetes Analyseband selbst anzugeben, womit eine automatisierte Bandfertigung ohne aufwändige Handhabungsschritte möglich ist.

Zur Lösung dieser Aufgabe wird die im Kennzeichenteil der unabhängigen Patentansprüche jeweils angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, den schichtweisen Bandaufbau durch einen zweistufigen Prozess zu vereinfachen. Dementsprechend wird in verfahrensmäßiger Hinsicht vorgeschlagen, dass ein Testetikettenband zumindest aus einem Nachweisfilm und einem Klebeband vorgefertigt wird, und dass die Testfelder als selbstklebende Testetiketten von dem Testetikettenband auf das Transportband übertragen werden. Der Etikettiervorgang erlaubt eine einfache Positionierung und Anbringung der Testfelder im Durchlauf des Transportbandes. Damit ist eine kontinuierliche Verarbeitung in einer Fertigungslinie möglich, ohne dass eine kinematisch komplexe Handhabung durch Pick-and-Place-Geräte erforderlich wäre.

Vorteilhafterweise wird eine Klebeverbindung der Testfelder dadurch hergestellt, dass ein doppelseitiges Klebeband an seiner einen Klebeseite mit dem Nachweisfilm und an seiner anderen Klebeseite (in Form der Selbstklebeetiketten) mit dem Transportband verbunden wird.

Um die Prozesssicherheit zu erhöhen, ist es günstig, wenn das Klebeband durch eine beidseitig mit Klebstoff beschichtete Zwischenfolie stabilisiert wird.

Eine weitere Verbesserung sieht vor, dass der Nachweisfilm unter Freihaltung von seitlichen Klebestreifen auf das Klebeband aufgebracht wird. Zur gleichmäßigen Probenverteilung kann der Nachweisfilm durch eine vorzugsweise als Gewebe oder Vlies ausgebildete Decklage überdeckt sein, wobei die Decklage vorteilhafterweise breiter als der Nachweisfilm ist und im Bereich ihrer überstehenden Ränder durch das Klebeband gehalten wird.

Um die erforderliche Probenmenge weiter reduzieren zu können, ist es von Vorteil, wenn die saugfähige Decklage außerhalb einer Nachweiszone mit einer wasserabweisenden Imprägnierung versehen wird.

Eine verfahrensmäßige Vereinfachung ergibt sich dadurch, dass die Imprägnierung durch ein Druckverfahren seitlich von dem Nachweisfilm streifenförmig aufgedruckt wird. Denkbar ist es auch, dass bereits die Fasern einer die Decklage bildende Filamentstruktur mit einem Imprägniermittel ummantelt werden.

Herstellungstechnisch ist es von besonderem Vorteil, wenn das Klebeband über eine Trägerbahn von Rolle zu Rolle verarbeitet wird. Dabei können die Testetiketten durch Schneiden oder Stanzen als lösbare Flächengebilde auf einer Trägerbahn des Klebebands erzeugt werden.

Die Zuführung im Durchlauf lässt sich dadurch realisieren, dass die Testetiketten von der Trägerbahn des Klebebandes vorzugsweise durch Umlenken über eine Spendekante abgelöst und auf das von Rolle zu Rolle transportierte Transportband aufetikettiert werden.

Für eine ökonomische Parallelverarbeitung ist es von Vorteil, wenn ein vielspuriges Testetikettenband durch mehrere Nachweisfilme nebeneinander auf einem Klebeband gebildet wird. Dabei kann das vielspurige Testetikettenband durch Schneiden oder Stanzen abschnittsweise in Vielfach-Testetiketten unterteilt werden. Alternativ ist es auch möglich, dass das vielspurige Testetikettenband durch Schneiden oder Stanzen abschnittsweise in Etikettenblöcke mit mehreren nebeneinander liegenden Einzel-Testetiketten unterteilt wird. Die Vielfach-Testetiketten oder die Etikettenblöcke lassen sich dann im Abstand voneinander auf das Transportband aufetikettieren, wobei anschließend das Transportband in Einzelspuren längsgeteilt wird.

Vorrichtungsmäßig wird die eingangs genannte Aufgabe durch eine von Rolle zu Rolle arbeitende Fördereinrichtung für ein aufrollbares Transportband und eine Etikettiereinrichtung zum Aufetikettieren von zumindest aus einem Nachweisfilm und einem Klebeband gebildeten Testetiketten an einer Etikettierstelle auf das vorbeilaufende Transportband gelöst.

Vorteilhafterweise umfasst die Etikettiereinrichtung eine der Etikettierstelle vorgeordnete Stanz- oder Schneidvorrichtung, insbesondere einen Laserschneider zum Unterteilen des mit dem Nachweisfilm versehenen Klebebands in selbstklebende Testetiketten.

Das solchermaßen aus dem Transportband als Trägerteil und den darauf befindlichen Testetiketten als Testfelder gebildete Analyseband lässt sich an einer Konfektionierstation in Verbrauchseinheiten abpacken.

Im Folgenden wird die Erfindung anhand der in der Zeichnung schematisch dargestellten Ausführungsbeispiele näher erläutert. Es zeigen
- Fig. 1: ein Analyseband mit einem mehrlagigen Testfeld in einer ausschnittsweisen perspektivischen Darstellung;
- Fig. 2: ein Testgerät mit dem darin eingesetzten Analyseband im Schnitt;
- Fig. 3 bis 6: verschiedene Aufbaustufen eines die Testfelder bildenden Testetikettenbands jeweils in der Draufsicht und im Querschnitt;
- Fig. 7: das in einzelne Testetiketten geschnittene Testetikettenband in ausschnittsweiser Draufsicht;
- Fig. 8: ein gestanztes Testetikettenband in einer Fig. 7 entsprechenden Darstellung;
- Fig. 9: eine Draufsicht des Analysebandes nach Fig. 1;
- Fig. 10 bis 13: verschiedene Schritte einer vielspurigen Fertigung von Analysebändem in ausschnittsweiser Draufsicht; und
- Fig. 14 bis 17: ein weiteres Ausführungsbeispiel einer Vielspur-Fertigung in einer Fig. 10 bis 13 entsprechenden Darstellung.

Die in der Zeichnung dargestellten Analysebänder 10 bestehen aus einem aufrollbaren Transportband 12 mit einer Vielzahl von darauf aufgebrachten, in Bandrichtung im Abstand voneinander befindlichen Testfeldem 14 zur Analyse von Körperflüssigkeiten, insbesondere Blut.

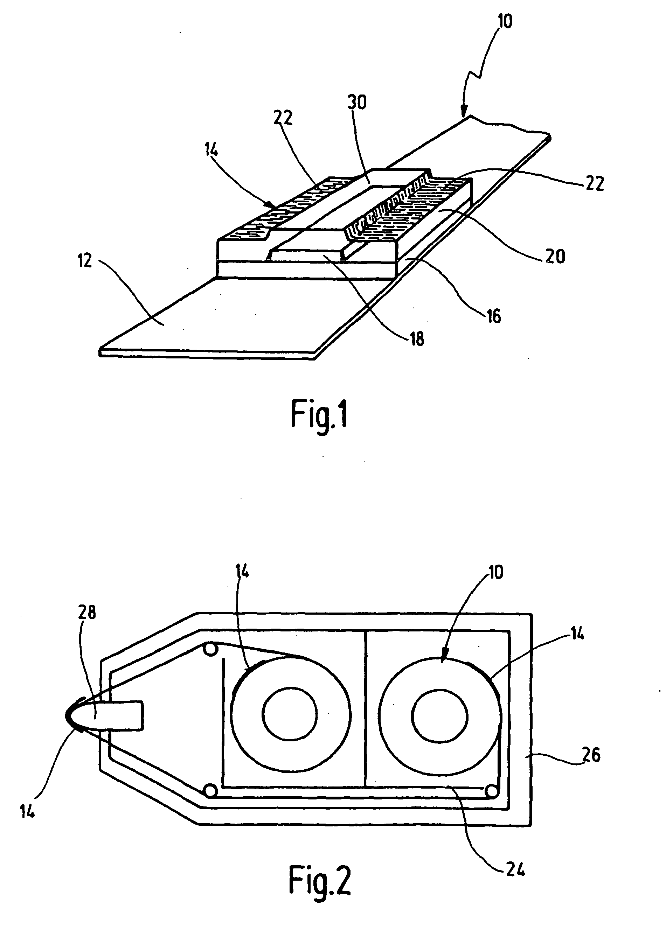

Wie aus Fig. 1 ersichtlich, sind die Testfelder 14 als selbstklebende Testetiketten mehrlagig ausgebildet. Sie umfassen jeweils einen Abschnitt eines Klebebandes 16, eines Nachweisfilms 18 und einer saugfähigen Decklage 20, welche bereichsweise mit einer Imprägnierung 22 versehen ist.

Gemäß Fig. 2 lässt sich das aufgerollte Analyseband 10 in Form einer Kassette 24 in ein tragbares Blutzucker-Testgerät 26 einsetzen. Durch Vorspulen können die einzelnen Testfelder bzw. Testetiketten 14 im Bereich eines Messkopfes 28 exponiert werden, um einen Bluttropfen für die Glukosebestimmung aufzutragen. Die Flüssigkeitsaufnahme erfolgt dabei in der zentralen Nachweiszone 30 der Decklage 20, wobei die imprägnierten Randstreifen 22 die Flüssigkeitsausbreitung begrenzen. Aufgrund des mehrlagigen Aufbaus besitzen die Testfelder 14 eine gewisse Höhe, während das dünne flexible Transportband 12 in den dazwischenliegenden Bereichen eine zuverlässige Abdichtung an Dichtelementen erlaubt, so dass eine sichere und vor Umwelteinflüssen geschützte Magazinierung möglich ist.

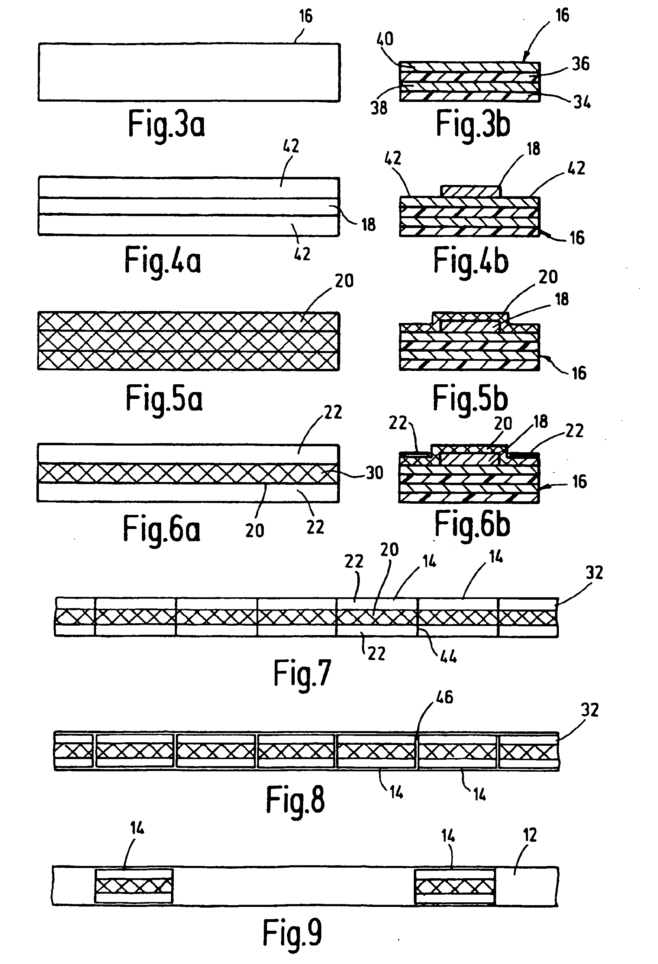

Die Herstellung des Analysebandes 10 erfolgt durch eine Rolle-zu-Rolle-Verarbeitung in zwei Verfahrensstufen. In der ersten Stufe gemäß Fig. 3 bis 8 wird zunächst ein Testetikettenband 32 vorgefertigt, woraufhin dann in der zweiten Stufe gemäß Fig. 9 die selbstklebenden Testetiketten 14 von dem Testetikettenband 32 auf das Transportband 12 übertragen werden.

Wie in der Draufsicht Fig. 3a und - nicht maßstäblich - im Querschnitt Fig. 3b veranschaulicht, bildet das doppelseitige Klebeband 16 die Basis des Testetikettenbands 32. Dieses besitzt eine Trägerbahn 34 bzw. einen Release-Liner für einen Bandtransport von einer Abwickelspule zu einer Aufwickelspule über verschiedene Verarbeitungsstationen. Auf der Trägerbahn 34 befinden sich zwei durch eine Zwischenschicht 36 getrennte Klebeschichten 38, 40. Entsprechend Fig. 4 wird auf der oberen freien Klebeschicht 40 des durchlaufenden Klebebands 16 ein Nachweisfilm 18 mit geringerer Breite zentriert aufgeklebt, so dass seitliche Klebestreifen 42 der Klebeschicht 40 freigehalten werden. Danach wird gemäß Fig. 5 die als Gewebe ausgebildete Decklage 20 bahnförmig aufgebracht. Die Decklage 20 ist breiter als der Nachweisfilm 18 und wird daher im Bereich ihrer seitlich überstehenden Ränder durch die freien Klebestreifen 42 fixiert. Die überstehenden Randstreifen der Decklage 20 außerhalb des Nachweisfilms 18 werden anschließend mit einer wasserabweisenden Imprägnierung 22 bedruckt, so dass nur die zentrale Nachweiszone 30 die aufzubringende Körperflüssigkeit aufsaugen und begrenzt ausbreiten kann (Fig. 6).

Auf dem solchermaßen hergestellten Testetikettenband 32 können nach Fig. 7 einzelne Testetiketten 14 als lösbare Flächengebilde freigeschnitten werden. Hierzu werden in Bandquerrichtung verlaufende Schnitte 44 beispielsweise durch Laserschneiden von der Decklage 20 her bis zur Tiefe der Trägerbahn 34 eingebracht, so dass die Testetiketten 14 mit der Klebeschicht 38 abgelöst werden können. Anstelle des Schneidvorgangs kann die Vereinzelung auch durch Stanzen entsprechend Fig. 8 erfolgen, wobei das Stanzgitter 46 unter Freistellung der Etiketten 14 von dem Etikettenband 32 abgezogen wird.

In der zweiten Verfahrensstufe wird die Transportbahn 12 von einer Abwickelrolle zu einer Aufwickelrolle geführt, während die Einzeletiketten 14 an einer Etikettierstation gemäß Fig. 9 im Abstand voneinander aufgebracht werden. Zu diesem Zweck werden die Testetiketten 14 von der Trägerbahn 34 des Klebebandes 16 durch Umlenken über eine Spendekante abgelöst und über die freigelegte Klebeschicht 38 auf das Transportband 12 selbstklebend aufgedrückt.

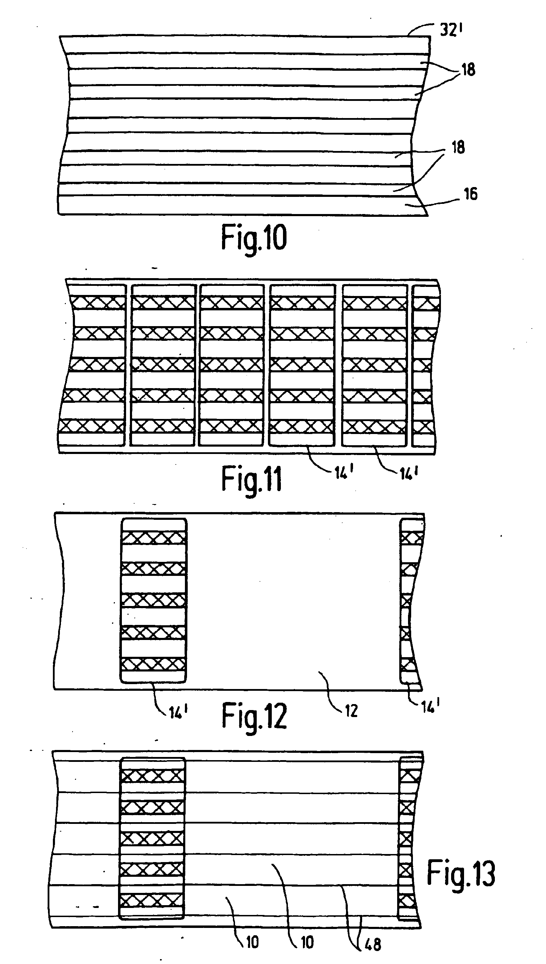

Das vorstehend beschriebene Verfahrensprinzip lässt sich für eine ökonomischere Herstellung auch in einem Vielspur-Prozess anwenden. Hierfür wird gemäß Fig. 10 ein vielspuriges Testetikettenband 32' in dem vorstehend beschriebenen Aufbau mit mehreren parallelen Nachweisspuren 18 auf einem breiten Klebeband 16 gebildet. Nach Fig. 11 wird dieses Vielspur-Etikettenband 32' durch Stanzen und Abziehen des Stanzgitters abschnittsweise in Vielfach-Testetiketten 14' unterteilt. Diese können entsprechend Fig. 12 im Abstand voneinander auf ein breites Transportband 12 aufetikettiert werden, woraufhin das Transportband gemäß Fig. 13 durch Längsschnitte 48 in Einzelspuren bzw. einzelne Analysebänder 10 geteilt wird.

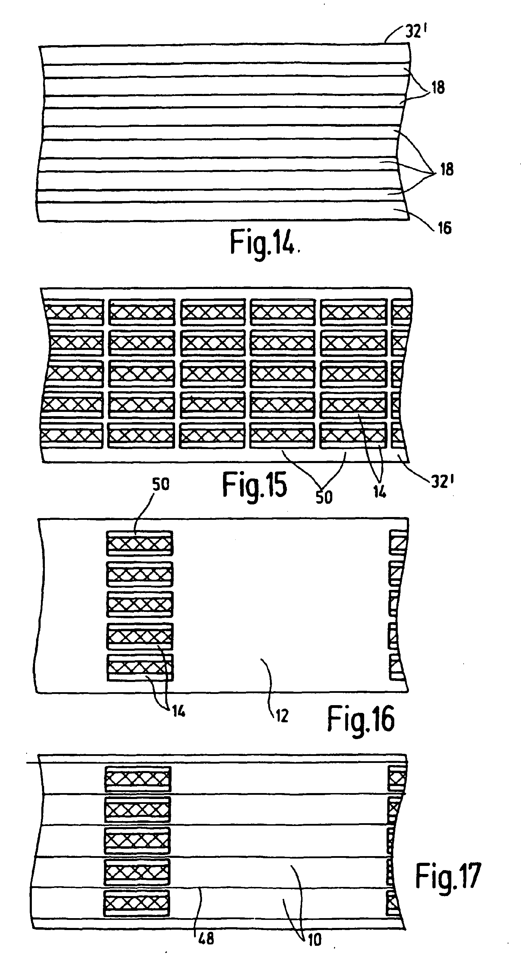

Das in Fig. 14 bis 17 veranschaulichte Ausführungsbeispiel unterscheidet sich im Wesentlichen nur dadurch, dass anstelle der Vielfach-Testetiketten 14' durch Stanzen abschnittsweise Etikettenblöcke 50 aus mehreren (vorliegend fünf) Einzel-Testetiketten 14 auf dem Trägerband 32' gebildet werden (Fig. 15). Diese können dann entsprechend Fig. 16 blockweise auf das Transportband 12 aufetikettiert werden, um wiederum die einzelnen Analysebänder 10 durch Längsschnitte 48 zu erhalten.

## Patentansprüche

1. Verfahren zur Herstellung eines Analysebandes (10) für Flüssigproben, insbesondere Körperflüssigkeiten, bei welchem ein aufrollbares Transportband (12) mit einer Vielzahl von in Bandrichtung im Abstand voneinander befindlichen Testfeldern (14) zur Analyse der Flüssigproben versehen wird, **dadurch gekennzeichnet, dass** ein Testetikettenband (32) zumindest aus einem Nachweisfilm (18) und einem Klebeband (16) vorgefertigt wird, und dass die Testfelder als selbstklebende Testetiketten (14) von dem Testetikettenband (32) auf das Transportband (12) übertragen werden.

2. Verfahren nach Anspruch 1, **dadurch gekennzeichnet, dass** ein doppelseitiges Klebeband (16) an seiner einen Klebeseite (40) mit dem Nachweisfilm (18) und an seiner anderen Klebeseite (38) mit dem Transportband (12) verbunden wird.

3. Verfahren nach Anspruch 1 oder 2, **dadurch gekennzeichnet, dass** das Klebeband (16) durch eine beidseitig mit Klebstoff beschichtete Zwischenfolie (36) stabilisiert wird.

4. Verfahren nach einem der Ansprüche 1 bis 3, **dadurch gekennzeichnet, dass** der Nachweisfilm (18) unter Freihaltung von seitlichen Klebestreifen (42) auf das Klebeband (16) aufgebracht wird.

5. Verfahren nach einem der Ansprüche 1 bis 4, **dadurch gekennzeichnet, dass** der Nachweisfilm (18) durch eine Decklage (20) für eine flächige Probenaufnahme überdeckt wird.

6. Verfahren nach Anspruch 5, **dadurch gekennzeichnet, dass** die Decklage (20) breiter als der Nachweisfilm (18) ist und im Bereich ihrer überstehenden Ränder durch das Klebeband (16) gehalten wird.

7. Verfahren nach Anspruch 5 oder 6, **dadurch gekennzeichnet, dass** die saugfähige Decklage (20) außerhalb einer Nachweiszone (30) mit einer wasserabweisenden Imprägnierung (22) versehen wird.

8. Verfahren nach einem der Ansprüche 5 bis 7, **dadurch gekennzeichnet, dass** ein Imprägnierungsmittel durch ein Druckverfahren seitlich von dem Nachweisfilm (18) streifenförmig aufgedruckt wird.

9. Verfahren nach einem der Ansprüche 5 bis 7, **dadurch gekennzeichnet, dass** eine die Decklage (20) bildende Filamentstruktur mit einem Imprägniermittel ummantelt wird.

10. Verfahren nach einem der Ansprüche 1 bis 9, **dadurch gekennzeichnet, dass** das Klebeband (16) über eine Trägerbahn (34) von Rolle zu Rolle verarbeitet wird.

11. Verfahren nach einem der Ansprüche 1 bis 10, **dadurch gekennzeichnet, dass** die Testetiketten (14) durch Schneiden oder Stanzen als lösbare Flächengebilde auf einer Trägerbahn (34) des Klebebands (16) erzeugt werden.

12. Verfahren nach einem der Ansprüche 1 bis 11, **dadurch gekennzeichnet, dass** die Testetiketten (14) von der Trägerbahn (34) des Klebebands (16) abgelöst und auf das von Rolle zu Rolle transportierte Transportband (12) aufetikettiert werden.

13. Verfahren nach einem der Ansprüche 1 bis 12, **dadurch gekennzeichnet, dass** ein vielspuriges Testetikettenband (32') durch mehrere Nachweisfilme (18) nebeneinander auf einem Klebeband (16) gebildet wird.

14. Verfahren nach Anspruch 13, **dadurch gekennzeichnet, dass** das vielspurige Testetikettenband (32') durch Schneiden oder Stanzen abschnittsweise in Vielfach-Testetiketten (14') unterteilt wird.

15. Verfahren nach Anspruch 13, **dadurch gekennzeichnet, dass** das vielspurige Testetikettenband (32') durch Schneiden oder Stanzen abschnittsweise in Etikettenblöcke (50) mit mehreren nebeneinander liegenden Einzel-Testetiketten (14) unterteilt wird.

16. Verfahren nach Anspruch 14 oder 15, **dadurch gekennzeichnet, dass** die Vielfach-Testetiketten (14') oder die Etikettenblöcke (50) im Abstand voneinander auf das Transportband (12) aufetikettiert werden, und anschließend das Transportband (12) in Einzelspuren längsgeteilt wird.

## Claims

1. Process for producing an analytical tape (10) for liquid samples and in particular body fluids in which a rollable transport tape (12) is provided with a plurality of test fields (14) that are spaced apart in the direction of the tape for analysing the liquid samples, **characterized in that** a test label tape (32) is prefabricated from at least a detection film (18) and an adhesive tape (16) and that the test fields are transferred as self-adhesive test labels (14) from the test label tape (32) onto the transport tape (12).

2. Process as claimed in claim 1, **characterized in that** one adhesive side (40) of a double-sided adhesive tape (16) is bonded to the detection film (18) and the other adhesive side (38) is bonded to the transport tape (12).

3. Process as claimed in claim 1 or 2, **characterized in that** the adhesive tape (16) is stabilized by an intermediate foil (36) which is coated on both sides with adhesive.

4. Process as claimed in one of the claims 1 to 3, **characterized in that** the detection film (18) is applied to the adhesive tape (16) while keeping lateral adhesive strips (42) free.

5. Process as claimed in one of the claims 1 to 4, **characterized in that** the detection film (18) is covered by a cover layer (20) for a planar sample uptake.

6. Process as claimed in claim 5, **characterized in that** the cover layer (20) is wider than the detection film (18) and is held by the adhesive tape (16) in the area of its protruding edges.

7. Process as claimed in claim 5 or 6, **characterized in that** the absorbent cover layer (20) is provided with a water-repellent impregnation (22) outside a detection zone (30).

8. Process as claimed in one of the claims 5 to 7, **characterized in that** an impregnation agent is printed on in a strip shape at the sides of the detection film (18) by a printing process.

9. Process as claimed in one of the claims 5 to 7, **characterized in that** a filament structure forming the cover layer (20) is coated with an impregnation agent.

10. Process as claimed in one of the claims 1 to 9, **characterized in that** the adhesive tape (16) is processed from roll-to-roll by means of a carrier sheet (34).

11. Process as claimed in one of the claims 1 to 10, **characterized in that** the test labels (14) are produced as detachable flat structures on a carrier sheet (34) of the adhesive tape (16) by cutting or punching.

12. Process as claimed in one of the claims 1 to 11, **characterized in that** the test labels (14) are detached from the carrier sheet (34) of the adhesive tape (16) and labelled onto the transport tape (12) as it is transported from roll-to-roll.

13. Process as claimed in one of the claims 1 to 12, **characterized in that** a multitrack test label tape (32') is formed by several detection films (18) next to one another on an adhesive tape (16).

14. Process as claimed in claim 13, **characterized in that** the multitrack test label tape (32') is divided in sections into multiple test labels (14') by cutting or punching.

15. Process as claimed in claim 13, **characterized in that** the multitrack test label tape (32') is divided in sections into label blocks (50) comprising several individual test labels (14) lying next to one another by cutting or punching.

16. Process as claimed in claim 14 or 15, **characterized in that** the multiple test labels (14') or the label blocks (50) are labelled spaced apart onto the transport tape (12) and subsequently the transport tape (12) is divided longitudinally into individual tracks.

## Revendications

1. Procédé pour fabriquer une bandelette d'analyses (10) d'échantillons liquides, notamment de liquides corporels, selon lequel une bande de transport enroulable (12) est dotée d'une multitude de champs de test (14) espacés les uns des autres dans la direction de la bande, pour analyser les échantillons liquides, **caractérisé en ce qu'**une bande d'étiquettes de test (32) est préfabriquée à partir d'un film de contrôle (18) et d'un ruban adhésif (16), et **en ce que** les champs de test sont transférés sur la bande de transport (12) à partir de la bande d'étiquettes de test (32).

2. Procédé selon la revendication 1, **caractérisé en ce qu'**un ruban adhésif double face (16) est doté sur une face adhésive (40) du film de contrôle (18) et sur l'autre face adhésive (38) de la bande de transport (12).

3. Procédé selon la revendication 1 ou 2, **caractérisé en ce que** le ruban adhésif (16) est renforcé par une feuille intercalaire (36) enduite de colle de part et d'autre.

4. Procédé selon l'une des revendications 1 à 3, **caractérisé en ce que** le film de contrôle (18) est appliqué sur le ruban adhésif (16) en laissant libres des bandes adhésives latérales (42).

5. Procédé selon l'une des revendications 1 à 4, **caractérisé en ce que** le film de contrôle (18) est recouvert d'une couche de recouvrement (20) destinée à recevoir un échantillon en nappe.

6. Procédé selon la revendication 5, **caractérisé en ce que** la couche de recouvrement (20) est plus large que le film de contrôle (18) et est maintenue par le ruban adhésif (16) dans la zone de ses bords qui dépassent.

7. Procédé selon la revendication 5 ou 6, **caractérisé en ce que** la couche de recouvrement absorbante (20) est dotée en dehors de la zone de contrôle (30) d'une imprégnation hydrophobe (22).

8. Procédé selon l'une des revendications 5 à 7, **caractérisé en ce qu'**un produit d'imprégnation est imprimé sous forme de bandes, par un procédé d'impression, de part et d'autre du film de contrôle (18).

9. Procédé selon l'une des revendications 5 à 7, **caractérisé en ce qu'**une structure filamenteuse formant la couche de recouvrement (20) est enrobée d'un produit d'imprégnation.

10. Procédé selon l'une des revendications 1 à 9, **caractérisé en ce que** le ruban adhésif (16) est transformé de rouleau à rouleau par l'intermédiaire d'une bande de support (34).

11. Procédé selon l'une des revendications 1 à 10, **caractérisé en ce que** les étiquettes de test (14) sont découpées ou poinçonnées dans une bande de support (34) du ruban adhésif (16) sous forme de structures plates amovibles.

12. Procédé selon l'une des revendications 1 à 11, **caractérisé en ce que** les étiquettes de test (14) sont décollées de la bande de support (34) du ruban adhésif (16) et apposées sur la bande de transport (12) transportée de rouleau à rouleau.

13. Procédé selon l'une des revendications 1 à 12, **caractérisé en ce que** l'on forme une bande d'étiquettes de test (32') à plusieurs pistes en collant plusieurs films de contrôle (18) côte à côte sur un ruban adhésif (16).

14. Procédé selon la revendication 13, **caractérisé en ce que** la bande d'étiquettes de test (32') à plusieurs pistes est débitée par découpage ou poinçonnage en étiquettes de test multiples (14').

15. Procédé selon la revendication 13, **caractérisé en ce que** la bande d'étiquettes de test (32') à plusieurs pistes est débitée par découpage ou poinçonnage en blocs d'étiquettes (50) comportant plusieurs étiquettes de test unitaires (14) situées côte à côte.

16. Procédé selon la revendication 14 ou 15, **caractérisé en ce que** les étiquettes de test multiples (14') ou les blocs d'étiquettes (50) sont collé(e)s de façon espacée les un(e)s des autres sur la bande de transport (12), et **en ce que** la bande de transport (12) est ensuite divisée longitudinalement en bandes individuelles.
